# EUROPÄISCHE PATENTANMELDUNG

(11) **EP 1 273 260 A1**
(43) Veröffentlichungstag der Anmeldung: **08.01.2003**
(21) Anmeldenummer: 01116028.0
(22) Anmeldetag: 02.07.2001
(51) Int. Cl.: A61B 5/00

(54) **Ohrläppchensensor**

(71) Anmelder: Trion AG, 8604 Volketswil (CH)
(72) Erfinder: Carlson, Sven-Erik, 8704 Herrliberg (CH); Zünd, Gregor, 8704 Herrliberg (CH)
(74) Vertreter: Troesch Scheidegger Werner AG

(57) **Zusammenfassung**

Für die Ueberwachung des Gesundheitszustandes einer Person bzw. zum Erfassen medizinischer Daten wird ein Ohrsensor vorgeschlagen, für die drahtlose Uebertragung der durch einen Sensor ermittelten oder gemessenen Werten an einen Dritten bzw. an einen Empfänger. Die Anordnung weist eine an einem Ohr platzierbare Vorrichtung auf, welche mindestens an je einer Seite des Ohrläppchens und/oder der Ohrmuschel platzierbar je eine Partie aufweist, eine Partie aufweisend ein Organ (9) für Lichttransmission und die andere Partie aufweisend einen Lichtsensor (7), zum pulsoxymetrischen Ermitteln des durch das Läppchens oder der Muschel absorbierten Lichtes. Schliesslich weist die Vorrichtung einen Sender (16) für die drahtlose Uebertragung de durch den Sensor (7) ermittelten Werte bzw. daraus abgeleiteter Auswertdaten auf, für die Uebertragung an den Empfänger.

## Beschreibung

Die vorliegende Erfindung betrifft eine Anordnung zum Ueberwachen des Gesundheitszustandes einer Person, Anwendungen der Anordnung sowie ein Verfahren zur Ueberwachung des Gesundheitszustandes einer Person.

Durch die kontinuierliche Gesundheitszustandsüberwachung wird eine Früherkennung eines anormalen Gesundheitszustandes und eine Frühalarmierung von Drittpersonen ermöglicht.

Eine etablierte Methode zur Ueberwachung von vitalen Parametern ist die Erfassung des Gesundheitszustandes mittels Pulsoxymetrie. Die Pulsoxymetrie erlaubt eine sofortige Invivo-(am Lebenden) Messung der arteriellen Sauerstoffsättigung durch die Bestimmung der Farbe des Blutes zwischen einer Lichtquelle und einer Fotodetektor. Dabei wird im Normalfall Licht in zwei verschiedenen Wellenlängen, wie beispielsweise 660 nm und 940 nm verwendet. Die Methode beruht auf der Lichtabsorption im durchstrahlten Gewebe, wobei die Lichttransmission umgekehrt proportional zur Konzentration des Hämoglobins ist. Während jedes Herzzyklus ändert sich die Lichtabsorption zyklisch: während der Diastole durch venöses Blut, Gewebe, Knochen und Pigment, während der Systole durch arterielles Blut, kapilläres Blut, venöses Blut, Knochen und Pigment.

Für pulsoxymetrische Messungen eignen sich Körperpartien, wie Finger, Zehen, Ohrläppchen und dgl., d.h. Partien wo eine Lichtabsorption visuell erfasst werden kann.

Veränderungen der Pulsoxymetrie kann eine Veränderung des Gesundheitszustandes bedeuten. Aus der Pulsoxymetriekurve lassen sich die Herzfrequenz, als auch die SauerstoffSättigung direkt ermitteln. Den Blutdruck und das Herzminuten-Volumen lassen sich aus der Kurve ableiten, wenn nicht direkt, dann zumindest relativ zum Normalzustand der überwachten Person.

Insbesondere lassen sich der Herzkreislauf-Zustand mittels Pulsoxymetrie überwachen, wobei dies sowohl an gesunden Personen erfolgen kann, wie auch an Personen, welche unter Herzkreislauf-Störungen leiden.

Messeinrichtungen für Pulsoxymetrie werden insbesondere in Spitälern zur Ueberwachung von Patienten auf den verschiedensten Gebieten eingesetzt. So beschreiben die US 4 685 464, WO 00/78209, WO 01/13790 und die WO 01/41634 clipsartige Einrichtungen, welche vorzugsweise an Fingern platziert werden, um mittels einer Lichtquelle und einem entsprechenden Sensor pulsoxymetrische Messungen zu ermöglichen.

Gemeinsam all dieser Einrichtungen ist, dass eine Kabelverbindung zwischen Sensor und Auswerteinheit besteht und dass die Auswerteinheiten verhältnismässig gross und umständlich sind. Deshalb ist eine ortsunabhängige, kontinuierliche Ueberwachung von sich frei bewegenden Personen nur sehr beschränkt möglich. Es ist aber wichtig, dass beispielsweise bei Patienten, welche aus dem Spital entlassen werden, bei nichthospitalisierten Personen, welche unter Herz/Kreislaufstörungen leiden, bei Personen, die einer Risikogruppe angehören, wie z.B. Personen mit positiver Familienananmnese für cardiovaskuläre Erkrankungen, oder andere Risikokonstellationen besitzen, aber auch für gesunde Leute, die eine optimale Ueberwachung ihrer Gesundheit bevorzugen, oder beispielsweise auch bei Hochleistungssportlern, deren Gesundheitszustand zu überwachen ist, ist eine bewegungsunabhängige, ortsunabhängige und kontinuierliche, nicht invasive Ueberwachung des- oder derselben möglich.

Es ist daher eine Aufgabe der vorliegenden Erfindung eine Anordnung vorzuschlagen, welche eine möglichst nicht invasive, kontinuierliche, bewgungsmässig-unabhängige und/oder ortsunabhängige Ueberwachung des Gesundheitszustandes einer Person ermöglicht.

Vorgeschlagen wird eine Anordnung gemäss dem Wortlaut, insbesondere nach Anspruch 1.

Die Anordnung zum Ueberwachen des Gesundheitszustandes einer Person ist gekennzeichnet durch
- eine, an einem Ohr platzierbare Vorrichtung, welche mindestens an zwei Seiten des Ohrläppchens und/oder der Ohrmuschel platzierbar je eine Partie aufweist,
- eine Partie, aufweisend ein Organ für Lichttransmission und
- die andere Partie, aufweisend einen Lichtsensor, zum Ermitteln des durch das Läppchen und/oder Ohrmuschel absorbierten Lichtes, sowie
- einen Sender für die drahtlose Uebertragung der durch den Sensor ermittelten Werte bzw. daraus abgeleiteter Auswertdaten an einen Empfänger.

Bei den im Stand der Technik beschriebenen Messeinrichtung handelt es sich in der Regel um solche, welche vorzugsweise an einem Finger einer Person, wie beispielsweise einem Patienten, platziert werden. Der grosse Nachteil von an Fingern platzierten Messeinrichtungen liegt darin, dass Werte, wie beispielsweise der Blutdruck, stark unterschiedlich sind, ob eine Hand nach unten hängend angeordnet ist oder beispielsweise über dem Kopf gehalten wird. Dies sind somit Störfaktoren, welche falsche Messwerte ergeben bzw. welche ein Auswerten der ermittelten Messwerte erschweren. Das Anordnen der Messeinrichtung am Ohrläppchen oder der Ohrmuschel ist aus diesem Grunde vorteilhaft, da Störfaktoren durch unterschiedliche Kopfhaltung und Bewegung wesentlich geringer sind. Aus diesem Grunde wird erfindungsgemäss vorgeschlagen, die Messelektronik an einem Ohrläppchen oder der Ohrmuschel anzuordnen, wobei es wesentlich ist, dass Messdaten ohne Kabelverbindung an einen Empfänger übermittelt werden können, um Bewegungs- und Ortsabhängigkeit der zu überwachenden Person zu ermöglichen.

Es versteht sich von selbst, dass eine derartige Sendeeinrichtung in Folge des Platzierens an einem Ohr bzw. im Bereich eines Ohrläppchens oder der Muschel möglichst klein auszubilden ist. Aus diesem Grunde wird erfindungsgemäss vorgeschlagen, dass die Uebertragung der durch den Sensor gemessenen Daten oder durch eine Auswerteinrichtung abgeleitete Daten mittels Radiofrequenz-Technologie erfolgt.

Vorzugsweise umfasst die erfindungsgemäss vorgeschlagene Anordnung eine Befestigungseinrichtung am Ohr, wie beispielsweise einen Bügel, eine Klammer, Klemme, eine durch das Ohr hindurch verlaufende Partie oder eine Klebverbindung. Es ist wesentlich, dass die Messsensorik stabil am Ohrläppchen oder der Muschel angeordnet ist, um eine kontinuierlich gleichbleibende Messung zu ermöglichen, und um die Störfaktoren möglichst zu minimieren. Weiter umfasst die Anordnung die Messsensorik am Ohrläppchen, wie eingangs erwähnt sowie gegebenenfalls eine Elektronik für die Signalverarbeitung und Signalanalyse. Schliesslich umfasst die Anordnung eine Batterie oder Solarzellen für die Stromversorgung sowie einen Sender im Radiofrequenzbereich und gegebenenfalls Empfänger für die Kommunikation mit einem externen Gerät zwecks Datenübertragung. Dabei kann es sich beim externen Gerät entweder direkt um den Empfänger handeln, welcher für die Ueberwachung des Gesundheitszustandes der Person zuständig ist, oder aber um eine weitere Sendeund Empfangseinrichtung für Sprache und/oder Daten zu einer weiteren externen Empfangszentrale, wie beispielsweise eine Alarmzentrale. Bei dieser zusätzlichen Sende- und Empfangseinrichtung kann es sich beispielsweise um ein mobiles Telekommunikationsgerät handeln, wie beispielsweise ein sogenanntes GSM-Telefon (Global System for Mobil communication), ein UMTS (Universal Mobil Telecommunication System), etc., welche Geräte allgemein üblich als drahtlose Kommunikationsmittel bzw. als Ersatz für stationäre Telefonate verwendet werden. Grundsätzlich können irgendwelche mobile Telekommunikationsgeräte verwendet werden, welche drahtlos Daten und/oder Sprachinformationen übertragen, sei dies via ein Telekommunikationsnetz oder via Internet.

Die erfindungsgemäss vorgeschlagene Anordnung eignet sich beispielsweise für die Ueberwachung von Personen mit Herz/Kreislaufstörungen, um beispielsweise einen Alarm bei einer Rettungszentrale zu generieren, falls die von der Sensoreinheit ermittelten Werte einen vorgegebenen Bereich verlassen bzw. falls Alarmgrenzen unter- oder überschritten werden. Weiter kann die Anordnung verwendet werden für gesunde Personen, welche eine erhöhte Sicherheit im täglichen Leben als wünschenswert erachten.

Eine weitere Anwendung ist die Herz/Rhythmusüberwachung in Verbindung bei einer medizinischen Abklärung. So kann beispielsweise bei einer periodisch stattfindenden medizinischen Ueberprüfung des Gesundheitszustandes, wie bei einem Checkup, der Arzt eine erwähnte Herz/Rhythmusüberwachung anordnen, wodurch die Person während einer gewissen Zeit, beispielsweise ein sogenanntes EKG-Gerät auf sich tragen muss. Dies ist umständlich und zudem kann nur gerade die Herzfrequenz gemessen werden. In diesem Falle ist es vorteilhaft, bei dieser Person den erfindungsgemäss vorgeschlagenen Ohrmesssensor anzuordnen, welcher einen hohen Tragkomfort aufweist, mit welchem zusätzliche Zustandsfaktoren gemessen werden können, und welcher eine einfache Aufzeichnung ermöglicht.

Wiederum eine weitere Anwendung ist die Ueberwachung von Säuglingen, um den plötzlichen Kindstod zu vermeiden, indem ein Alarm durch die Messsensorik bei den Eltern/Betreuern generiert wird.

Wiederum eine weitere Anwendung der erfindungsgemäss vorgeschlagenen Anordnung liegt bei der Ueberwachung von Sportlern, indem Messwerte zwecks Leistungsausweise, kontinuierlich übermittelt werden können und dementsprechend zentral ausgewertet werden. Natürlich ist auch eine Ueberwachung von Sportlern im obengenannten Sinne, d.h. zur Ueberwachung des Herz/Kreislaufsystems möglich.

Bei der obigen Liste handelt es sich selbstverständlich nur um Beispiele und sie ist keinesfalls abschliessend.

Es ist denkbar die Messsensoreinheit am Ohrläppchen zu ergänzen, um weitere kontinuierliche Messungen zu ermöglichen, wie den PCO₂ (CO₂-Sättigungsgrad im Blut) als auch den Blutzuckergehalt, die Blutverdünnung, den Hämatokrit und das Hämoglobin zum ermitteln bzw. zu berechnen.

Die Auswertung der Sensorsignale sowie die daraus resultierende Kurve und die Weitersendung der Resultate erfolgt mittels einer Signalverarbeitungs- und Signalanalyse-Einrichtung und eines Sendeapparates, welcher beispielsweise mittels Ohrbügel hinter der Ohrmuschel platziert wird. Für die drahlose Uebertragung der Daten wird vorzugsweise, wie bereits oben erwähnt, Datenkommunikation im Radiofrequenzbereich verwendet, wie beispielsweise die neuerdings für lokale Sprach- und Datenkommunikation verwendete, sogenannte "Bluetooth"- Technologie, welche auf einfachste Art und Weise und unter Verwendung kleinster Module einen drahtlosen Informationsaustausch zwischen mehreren Geräten ermöglicht.

Durch die Messungen am Ohrläppchen sind sehr gute Messresultate mit wenig Störungen zu erwarten, da diese Messungen wenig sensitiv zu Körperbewegungen sind, und nur einen kleinen Standard-Fehler (Abstand Herz/Ohrläppchen) beinhalten.

Die Erfindung wird nun beispielsweise und unter Bezug auf die beigefügten Figuren näher erläutert.

Dabei zeigen:
- Fig. 1: schematisch eine erfindungsgemässe Anordnung für Pulsoxymetrie-Messung an einem Ohr,
- Fig. 2: in Perspektive eine mögliche Ausgestaltung einer erfindungsgemässen Anordnung an einem Ohr,
- Fig. 2a: einen Ausschnitt aus Figur 2,
- Fig. 3: anhand einer schematischen Darstellung ein Beispiel einer möglichen Ueberwachung des Gesundheitszustandes einer Person, und
- Fig. 4: erneut anhand einer schematischen Darstellung die Ueberwachung des Gesundheitszustandes eines Sportlers mittels der erfindungsgemäss definierten Anordnung.

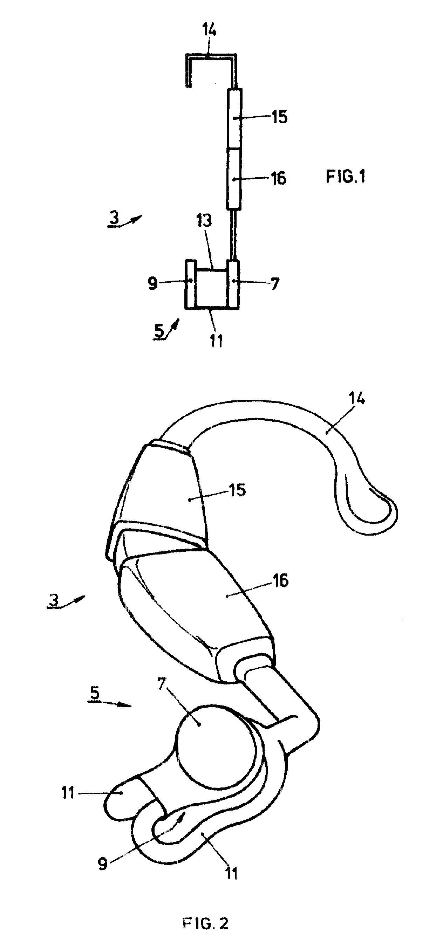

Figur 1 zeigt schematisch vereinfacht eine erfindungsgemässe Anordnung, vorgesehen um am Ohr der zu überwachenden Person angeordnet zu werden.

Die Messsensoreinheit 3 umfasst den eigentlichen Messsensor 5, bestehend aus einer Lichtquelle 9 und einem Fotodetektor 7, welche auf je einer Seite eines Ohrläppchens angeordnet werden, welche beispielsweise über eine bügelartige Verbindung 11 miteinander verbunden sind. Zur Fixierung der beiden Elemente 7 und 9 am Ohrläppchen kann es vorteilhaft sein, zusätzlich ein durch das Ohrläppchen hindurch verlaufende stiftartige Verbindung 13 vorzusehen, damit der Messsensor unverrückbar und positionstreu am Ohrläppchen angeordnet ist. Selbstverständlich kann diese Positionstreue auch erreicht werden durch die Verwendung einer Klemme, einer Klammer, durch Kleben der Elemente 7 und 9 am Ohr, etc.

An einem Ohrbügel 14, welcher sich mindestens teilweise um die Ohrmuschel herum erstreckt, sind weiter ein Sender/Empfänger 16 vorgesehen, sowie eine Batterie 15. In der Sende/Empfangseinheit 16 kann weiter eine Datenverarbeitungseinheit vorgesehen sein, in welcher die durch den Messsensor 7 ermittelten Daten aufgearbeitet bzw. ausgewertet werden können. Schliesslich ist es auch möglich, in dieser Datenverarbeitungseinheit Vorgabewerte bzw. Wertebereich für die zu messenden Faktoren, wie Blutdruck, Sauerstoffsättigung, etc. einzugeben, wobei bei Ueber- oder Unterschreiten der angegebenen Bereich ein entsprechendes Alarmsignal generiert wird. Bei der Sende/Empfangseinheit handelt es sich um eine im Radiofrequenz-operierende Einheit, d.h. die Uebertragung der Daten erfolgt im Radiofrequenzbereich.

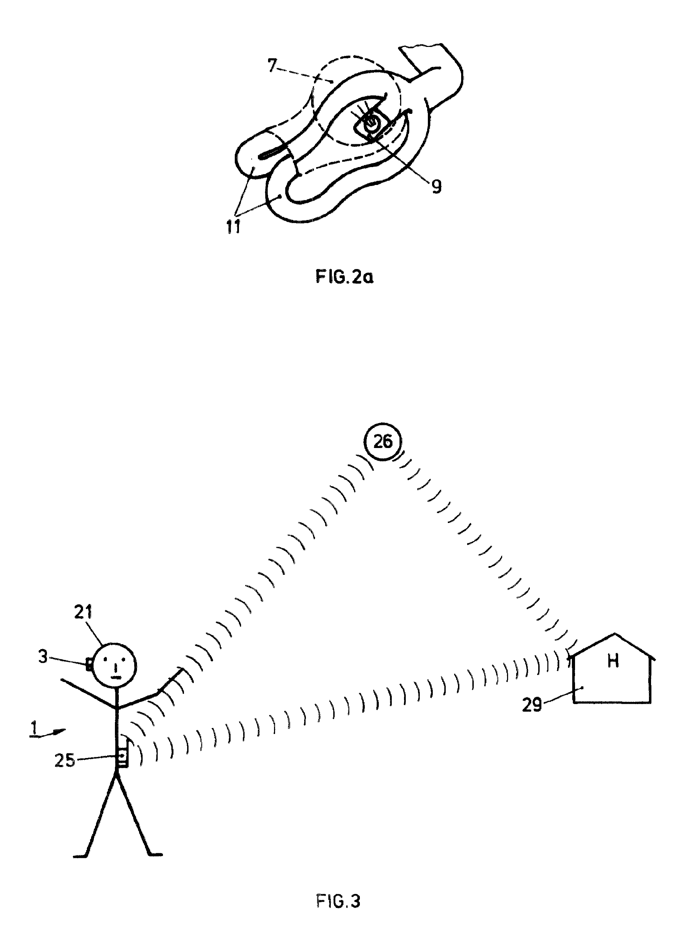

In Figur 2 ist eine konkretere Ausführungsvariante der Anordnung gemäss Figur 1 in Perspektive dargestellt, vorgesehen um an einem Ohr der zu überwachenden Person angeordnet zu werden. Wiederum umfasst die Anordnung 3 die Messsensoreinheit 5, umfassend eine Lichtquelle 9 (nicht sichtbar) sowie den Messsensor 7. Für die Fixierung und Verbindung der beiden Elemente 7 und 9 ist weiter eine Positionierungs-Vorrichtung 11, wie beispielsweise ein Klemmbügel 11, vorgesehen. Am Ohrbügel 14 angeordnet sind erneut die Batterie-Einheit 11 sowie die RF-Sende-/Empfangseinheit und Datenverarbeitungseinheit 16. Die Funktionsweise der Messsensorik beruht auf der Lichtabsorption im durchstrahlten Gewebe im Ohrläppchen, wobei die Lichttransmission umgekehrt proportional zur Konzentration des Hämoglobins ist. Während jedes Herzzyklus ändert sich die Lichtabsorption zyklisch. Aufgrund der schnellen Resorptionszeit und der Zuverlässigkeit der Messungen eignet sich ein Ohrläppchen am besten für pulsoxymetrische Messungen. Die Messung der arteriellen Sauerstoffsättigung ergibt sich durch die Bestimmung der Farbe des Blutes zwischen der Lichtquelle und dem Fotodetektor 7.

Figur 2a zeigt einen Ausschnitt aus Figur 2, wobei auf die Darstellung des Messsensors 7 weitgehendst verzichtet worden ist. Durch das Weglassen des Sensors ist die in Figur 2 nicht sichtbare Lichtquelle 9 erkennbar.

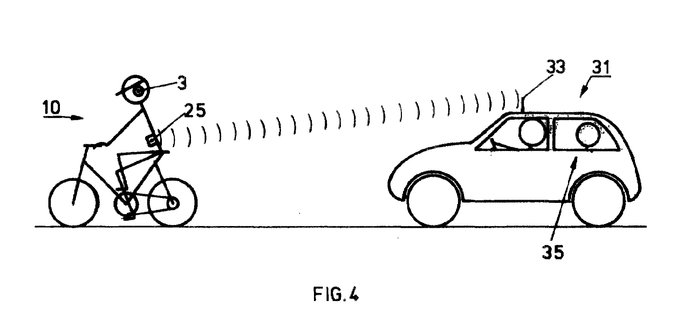

Anhand der Figuren 3 und 4 soll schematisch dargestellt werden, wie die durch die Messanordnung 3 erfassten bzw. ausgewerteten Daten an einen Empfänger übertragen werden können bzw. durch diesen ausgewertet werden können.

Bei einer Person 1 kann es sich beispielsweise um eine Person mit Herz/Kreislaufstörungen handeln. Diese kann sich beispielsweise in ärztlicher Behandlung befinden, oder aber es kann sich um eine Person handeln, welche erst kürzlich aus einem Spital entlassen worden ist, in welchem sie sich aufgrund beispielsweise eines Herzinfarktes aufgehalten hat. Mit anderen Worten kann bei der Person 1 der Verdacht bestehen, dass kurzfristig Herzprobleme auftreten können, welche für die Person 1 eine ernsthafte Bedrohung darstellen. Aus diesem Grunde ist es wichtig, dass die Person 1 ständig unter ärztlicher Kontrolle steht, d.h. das ständig der Gesundheitszustand der Person 1 überwacht werden kann.

Dies geschieht nun mit der Sensoranordnung 3, welche den unter Bezug auf die Figuren 1 und 2 beschriebenen Sensor 5 aufweist, mittels welchem die Herzfrequenz, der Blutdruck, die Sauerstoffsättigung, die Körpertemperatur, etc. überwacht werden können. In der in den Figuren 1 und 2 dargestellten Einheit 16 ist eine Logiksteuerung angeordnet, welche ständig kontrolliert, ob sich die Messwerte innerhalb oder ausserhalb eines durch einen Arzt des Patienten definierten Normalbereiches befinden. Werden Messwerte ausserhalb des Normalbereiches festgestellt, gibt der RF-Sender mittels einer drahtlosen Verbindung im Radiofrequenzbereich einen Befehl, beispielsweise an ein Mobiltelefon 25 ab, welches sich ebenfalls auf dem Patienten befindet. Aufgrund dieses Signals wird beim Mobiltelefon, bei welchem es sich beispielsweise um ein sogenanntes GSM-Telefon (Global System for Mobile communication) handelt, ein Wählimpuls ausgelöst, mittels welchem ein oder mehrere Empfänger angewählt werden. Beim Empfänger kann es sich beispielsweise um eine Rettungszentrale 29 handeln, welche beispielsweise von einer medizinischen Fachperson bedient wird. Bei Verbindungsaufbau durch die medizinische Fachperson werden dieser über die Verbindung vom Mobiltelefon 25 zum Anschluss im Spital 29, wie eine Telefonstation oder ein Internetanschluss, die von der Messeinheit gemessenen Daten übermittelt, so dass die Fachperson aufgrund dieser Daten und der Identifikation des Patienten, welcher ebenfalls durch das Mobiltelefon 25 ermöglicht wird, sofort eine Beurteilung des Gesundheitszustandes erstellen kann, und welche Massnahmen einzuleiten sind.

Wichtig kann ebenfalls sein, dass der medizinischen Fachperson die Positionskoordinaten des Patienten 1 bekannt sind, damit sie weiss, wo sich dieser aufhält. Dies lässt sich beispielsweise mittels des sogenannten und bereits weit verbreiteten GPS-Systems (Global Positioning System) feststellen, indem vom Mobiltelefon 25 zusätzlich zur Datenübertragung auch die Positionskoordinaten via Satellit 26 mittels des erwähnten GPS-Systems übertragen werden. Nun kann im Rettungszentrum entschieden werden, ob eine Equipe des Spitals oder eine auswärtige Stelle aufzubieten ist, um beim Patienten die notwendige Hilfe zu leisten.

Selbstverständlich handelt es sich in Figur 3 lediglich um ein Beispiel und die erfindungsgemäss vorgeschlagene Anordnung ist keineswegs auf die Ueberwachung von Patienten beschränkt, sondern auch die Ueberwachung des Gesundheitszustandes von gesunden Personen ist denkbar. Ein derartiges Beispiel soll anhand von Figur 4 näher erläutert werden.

In Figur 4 geht es um die Ueberwachung des Gesundheitszustandes bzw. um die Erfassung medizinischer Daten eines Velofahrers 10, um beispielsweise die Leistungsfähigkeit des Velofahrers zu ermitteln, um Trainingsmethoden zu optimieren, um den für den Velofahrer 10 optimalen Fahrstil zu eruieren, um generell medizinische Daten von aktiven Menschen zu ermitteln, etc.

Wiederum befindet sich die erfindungsgemäss vorgeschlagene Messanordnung 3 am Ohr des Velofahrer 10, welcher sich auf einer Velofahrt befindet. Erneut werden die vom Messsensor gemessenen bzw. ermittelten Werte an eine drahtlose Sendeeinrichtung 25 übertragen, wobei diese Datenübertragung vom Messsensor zur Datenübertragungseinheit 25 ebenfalls drahtlos im Radiofrequenzbereich erfolgt, beispielsweise mittels der sogenannten "Bluetooth"-Technologie. Von der Datensendeeinrichtung 25 werden die Daten drahtlos an beispielsweise eine Empfangsantenne 33 an einem Begleitfahrzeug 31 übermittelt, in welcher durch eine zuständige Person 35 die Daten ständig überwacht werden. Dabei kann es sich um eine medizinische Fachperson, um einen Trainer, oder ganz einfach um einen Bekannten des Velofahrers 10 handeln. Selbstverständlich ist es nicht zwingend notwendig, dass eine Person im Begleitfahrzeug 31 anwesend ist, indem die drahtlos übertragenen Daten auch aufgezeichnet bzw. abgespeichert werden können, um dann später ausgewertet zu werden. Selbstverständlich ist das Anordnen der Datenübertragungseinheit 25 nicht notwendig, falls der Sender der Messeinrichtung 3 genügend stark ist bzw. die Reichweite derart ist, dass der Empfänger 31 bzw. 33 erreicht werden kann. Schliesslich ist es aber auch möglich, direkt bei der Person 10 eine Datenaufzeichnungseinheit vorzusehen, auf welcher die Daten gespeichert werden, welche dann später bei Rückkehr des Velofahrers zu einem Trainingscenter oder nach Hause ausgewertet werden können bzw. auf eine grössere Datenverarbeitungseinheit übertragen werden können.

Selbstverständlich handelt es sich bei den beiden Situationen, dargestellt in den Figuren 3 und 4, lediglich um Beispiele, welche dazu geeignet sind, die vorliegende Erfindung näher zu erläutern. Die erfindungsgemäss vorgeschlagene Anordnung kann für x-beliebige andere Situationen verwendet werden, wo der Gesundheitszustand einer Person überwacht werden muss, bzw. wo medizinische Daten einer Person zu erfassen sind. Wie bereits oben erwähnt, ist es vorteilhaft, diese Messsensorik an einem Ohr anzuordnen. Der Vorteil liegt aber auch darin, dass die Messsensorik am Ohr in einen Gegenstand des täglichen Gebrauches integriert werden kann, wie beispielsweise in ein Hörgerät, in einen Ohrschmuck, in eine Kommunikationseinrichtung, wie beispielsweise bei Schauspielern, Sportlern, etc. verwendet, wo über das Ohr Instruktionen einer Person mitgeteilt werden.

## Patentansprüche

1. Anordnung zum Ueberwachen des Gesundheitszustandes einer Person bzw. zum Erfassen medizinischer Daten einer Person, **gekennzeichnet durch**
- eine an einem Ohr platzierbare Vorrichtung, welche mindestens an je einer Seite des Ohrläppchens und/oder der Ohrmuschel platzierbar je eine Partie aufweist,
- eine Partie aufweisend ein Organ (9) für Lichttransmission und
- die andere Partie aufweisend einen Lichtsensor (7), zum Ermitteln des **durch** das Läppchens oder der Muschel absorbierten Lichtes, sowie
- einen Sender (16) für die drahtlose Uebertragung der **durch** den Sensor (7) ermittelten Werte bzw. daraus abgeleitete Auswertdaten an einen Empfänger.

2. Anordnung nach Anspruch 1, **dadurch gekennzeichnet, dass** es sich beim Sender (16) um einen Sender im Radiofrequenzbereich handelt.

3. Anordnung nach einem der Ansprüche 1 oder 2, **dadurch gekennzeichnet, dass** die Vorrichtung eine Elektronik (16) bzw. eine Signalverarbeitungs- und Signalanalyse-Einrichtung für die Analyse bzw. Auswertung der durch den Sensor (7) ermittelten Werte aufweist.

4. Anordnung nach einem der Ansprüche 1 - 3, **dadurch gekennzeichnet, dass** die Vorrichtung eine Batterie (15) und/oder Solarzellen aufweist für die Stromversorgung.

5. Anordnung nach einem der Ansprüche 1 - 4, **dadurch gekennzeichnet, dass** eine Sende/Empfangseinrichtung (16) vorgesehen ist.

6. Anordnung nach einem der Ansprüche 1 - 5, **dadurch gekennzeichnet, dass** eine Elektronik für Analyse bzw. Auswertung der ermittelten Werte vorgesehen ist, weiter umfassend eine Logiksteuerung für das Feststellen von Unregelmässigkeiten der durch den Messsensor (7) erfassten Daten.

7. Anordnung nach einem der Ansprüche 1 - 6, **dadurch gekennzeichnet, dass** eine Zusatzsende- und Empfangseinrichtung (25) für Sprache und/oder Daten vorgesehen ist, um mindestens einen Dritten (29, 31) anzuwählen und an diesen Daten zu übertragen.

8. Anordnung nach Anspruch 7, **dadurch gekennzeichnet, dass** ein Ortungssystemmodul an der Sende/Empfangseinrichtung (25) vorgesehen ist, mittels welchem der Standort der Person an den Dritten übermittelt wird.

9. Verfahren zum Ueberwachen des Gesundheitszustandes einer Person bzw. zum Erfassen medizinischer Daten einer Person, **dadurch gekennzeichnet, dass** die Erfassung des Gesundheitszustandes bzw. der medizinischen Daten mittels Pulsoxymetrie am Ohrläppchen erfolgt, indem von einem Organ für Lichttransmission (9) Licht in mindestens zwei verschiedenen Wellenlängen durch das Ohrläppchen hindurch emittiert wird, dieses Licht durch einen Fotodetektor (7) erfasst wird, durch Messen der durch das durchstrahlte Gewebe im Ohrläppchen absorbierten Lichtes, die vom Fotodetektor (7) gemessen Werte an einen Sensor und gegebenenfalls an eine Auswertelektronik (16), welche ebenfalls im Bereich des Ohres angeordnet ist, übertragen wird und vom Sender (16) drahtlos im Radiofrequenzbereich an einen Empfänger übertragen wird.

10. Verfahren nach Anspruch 9, **dadurch gekennzeichnet, dass** die Uebertragung vom Sender (16) an einen Empfänger mittels "Bluetooth"-Technologie oder einer anderen propäritären Radiofrequenz-Uebertragungstechnik erfolgt.

11. Verfahren nach Anspruch 9 oder 10, **dadurch gekennzeichnet, dass** vom Fotodetektor erfasste Werte bzw. gegebenenfalls von der Auswertelektronik ermittelte Daten über den Sender (16) zunächst an eine weitere Sende- und Empfangseinrichtung für Sprache und/oder Daten (25) übertragen wird, von welcher aus ein Dritter (29) angewählt wird, um Daten zu übertragen.

12. Hörgerät mit einer Anordnung nach einem der Ansprüche 1 - 8.

13. Ohrschmuck bzw. Ohranhänger mit einer Anordnung nach einem der Ansprüche 1 - 8.

14. Gerät für Uebertragung von Anweisungen, Direktiven und dgl. im Bereich Sport, Unterhaltung, etc., vorgesehen für das Tragen im Bereich des Ohres mit einer Anordnung nach einem der Ansprüche 1 - 8.

15. Verwendung der Anordnung zur Ueberwachung einer Person mit Herz/Kreislaufstörungen.

16. Verwendung der Anordnung nach einem der Ansprüche 1 - 8, zum Erfassen sportmedizinischer Daten.

17. Verwendung der Anordnung nach einem der Ansprüche 1 - 8 für die Ueberwachung der Gesundheit von Personen, die eine cardiovaskuläre Risikokonstellation besitzen.
